# EUROPEAN PATENT SPECIFICATION

(11) **EP 2 111 556 B1**
(45) Date of publication and mention of the grant of the patent: **02.09.2015**
(21) Application number: 08702342.0
(22) Date of filing: 31.01.2008
(51) Int. Cl.: G01N 33/86

(54) **CONTAINER COMPRISING A DIAGNOSTIC COMPOSITION AND ITS USE IN THE DETERMINATION OF COAGULATION CHARACTERISTICS OF A TEST LIQUID**
BEHÄLTER MIT DIAGNOSTISCHER ZUSAMMENSETZUNG UND SEINE VERWENDUNG BEI DER BESTIMMUNG VON GERINNUNGSEIGENSCHAFTEN EINER TESTFLÜSSIGKEIT
RÉCIPIENT COMPRENANT UNE COMPOSITION DE DIAGNOSTIC ET SON UTILISATION POUR LA DÉTERMINATION DES CARACTÉRISTIQUES DE COAGULATION D'UN LIQUIDE D'ESSAI

(30) Priority: 01.02.2007 GB 0701821
(43) Date of publication of application: 28.10.2009
(60) Divisional application: 15177115.1
(73) Proprietor: DSM IP Assets B.V., 6411 TE Heerlen (NL)
(72) Inventor: CALATZIS, Andreas, 80469 München (DE); GLAUNER, Martin, 79639 Grenzach-Wyhlen (DE); SCHUBERT, Axel, 81927 München (DE); KESSLER, Max, 81539 München (DE)
(74) Representative: Seibel-Thomsen, Nadja
(86) International application number: PCT/IB2008/000208
(87) International publication number: WO 2008/093216

(56) References cited:
- EP-A- 1 367 135
- EP-A- 1 627 725
- WO-A-98/49563
- WO-A-02/079375
- US-A- 5 504 011

## Description

### FIELD OF THE INVENTION

The present invention is directed to a container comprising diagnostic composition for use in the viscoelastic analysis of a test liquid. The present invention further is directed to a method of performing a viscoelastic analysis on a test liquid, and to the use of the container in such a method.

### BACKGROUND

The coagulation of blood is a complex process during which blood forms solid clots. It is an important part of hemostasis (the cessation of blood loss from a damaged vessel) whereby a damaged blood vessel wall is covered by a blood clot to stop hemorrhage and aid repair of the damaged vessel. Disorders in coagulation can lead to increased hemorrhage and/or thrombosis and embolism.

In a normal individual, coagulation is initiated within 20 seconds after an injury occurs to the blood vessel damaging the endothelial cells. Platelets immediately form a haemostatic plug at the site of injury. This process is called primary haemostasis. Secondary haemostasis follows if plasma components called coagulation factors respond in a complex cascade to form fibrin strands which strengthen the platelet plug.

The coagulation cascade of secondary hemostasis has two pathways, the Contact Activation pathway (formerly known as the Intrinsic Pathway) and the Tissue Factor pathway (formerly known as the Extrinsic pathway) that lead to fibrin formation. It was previously thought that the coagulation cascade consisted of two pathways of equal importance joined to a common pathway. It is now known that the primary pathway for the initiation of blood coagulation is the Tissue Factor pathway. The pathways are a series of reactions, in which a zymogen of a serine protease and its glycoprotein co-factor are activated to become active components that then catalyze the next reaction in the cascade. Coagulation factors are generally indicated by Roman numerals from I - XIII, with a lowercase 'a' appended to indicate the activated form.

Fibrinolysis is the process where the fibrin clot is broken down. Tissue plasminogen activator (tPA) and urokinase are the agents that convert plasminogen to active plasmin, thus allowing fibrinolysis to occur.

The detection of normal or decreased functionality of these coagulation components is important in order to assess patients' hemostasis disorders.

Several methods of measuring the coagulation characteristics of blood are known. Some such devices attempt to simulate the natural flow of blood in the veins and arteries of a living subject, while other measurement techniques are performed in static blood volumes.

An accurate measurement of the ability of a patient's blood to coagulate in a timely and effective fashion is crucial to certain surgical and medical procedures. Rapid and accurate detection of abnormal coagulations is also of particular importance with respect to appropriate treatment to be given to patients suffering from clotting disorders. Often the condition of such patients makes it necessary to administer blood components, anti-coagulants, certain fibrinolytic agents, anti-platelet agents, or compounds inducing the reverse effects of said agents. In these cases, the treatment dose can be adapted to the extent of a clotting disorder previously determined.

Measurements of blood clotting are provided by various devices, for example as disclosed in (US 5,777,215), (US 6,537,819), or (US 5,777,215). These devices measure the mechanical properties of the clot throughout its structural development. These systems are summarized under the term "viscoelastic methods", as they continuously detect viscoelastic properties of the blood clot while its formation and lysis.

A viscoelastic measurement provides information about several distinct parameters, for example the time between coagulation activation and clot initiation (clotting time CT), the dynamics of clot formation (clot formation time CFT), the firmness of the clot (amplitudes A5-A30 and maximum clot firmness MCF), or the extent of fibrinolysis (maximum lysis ML).

A number of references describe instruments for measuring blood clotting characteristics based upon mechanical movements. These instruments monitor the elastic properties of blood as it is induced to clot under a low shear environment, i.e. in static blood volumes. The patterns of change in shear elasticity enable the determination of the kinetics of clot formation, as well as the strength and stability of the formed clot. The strength and stability of the clot provide information about the ability of the clot to perform the "work of hemostasis" (i.e., stop or prevent abnormal bleeding) and about the adequacy of blood platelet-fibrin interaction. The kinetics of clot formation mainly provides information about the functionality of coagulation factors. Analysis of all of this information provides results which are useful to predict bleeding, to monitor and manage thrombosis, or to monitor fibrinolysis.

However, as the clotting process consists of various interlinked components, the use of specific activators and inhibitors is further applied in order to detect hemostasis disorders more specifically.

Accordingly, the reagents used in viscoelastic analysis consist of an initial activator (e.g., an activator of either the intrinsic or the extrinsic pathway) and optionally one or more inhibitors (e.g., fibrinolysis inhibitors, heparin inhibitors, platelet inhibitors) and/or one or more further specific factor of the coagulation cascade.

Optionally further components may be added:
- Calcium (CaCl₂): The calcium is added for recalcification of the sample. Blood samples can be prevented from clotting by several different anticoagulatory substances like heparin, EDTA, citrate. Typically functional tests are done with blood anticoagulated with citrate. Citrate moderately complexes calcium of the blood sample. Calcium is necessary for the coagulation process, it is involved in complex formation and is co-factor for most of the coagulation factors (e.g., FI, FII, FV, FVII, FVIII, FIX, FX, FXI, FXIII, TF). Therefore, recalcification of the sample is necessary to ensure correct coagulation in the sample, if the sample was citrated during blood withdrawal (by using a blood tube containing citrate).
- Phospholipids: Several complexes in the coagulation cascade are phospholipid-dependent and, therefore, additional phospholipids might be added.
- Stabilizers: For the stabilization of the reagents between the time of production and the analysis (e.g. albumin, gelatine)

Depending on the diagnostic aim, these reagents can be used either alone or in combination: For example, a measurement with only intrinsic activator in the sample can be combined with a measurement with intrinsic activator and a sufficient amount of heparin inhibitor (e.g., heparinase) in the sample to detect the presence of heparin in the test liquid; a combination of extrinsic activator and platelet inhibitor (e.g., Cytochalasin D) in the sample is applied to determine the activity of fibrinogen without platelet contribution in the test liquid.

There is a reagent concept for viscoelastic measurements in the literature (ReoPro-modified TEG: Wenker et al.: Thrombelastography, The Internet Journal of Anesthesiology, 2000, Volume 1 Number 3; (http://www.ispub.com/ostia/index.php ?xmlFilePath=journals/ija/volln3/teg.xml); Ruttmann et al.: Hemodilution Enhanced Coagulation Is Not Due to Platelet Clumping, Anesthesiology 2004; 101: A150; Recombiplastin- and ReoPro-modified TEG:http://www.transfusionguidelines.org.uk/ docs/pdfs/bbt-app-use_teg-sop-example.pdf; TF- and Trasylol-modified TEG: Tanaka et al.: Evaluation of a novel kallikrein inhibitor on hemostatic activation in vitro, Thrombosis Research, Volume 113, Issue 5 , 2004, Pages 333-339) that is based on the combination of commercially available activator reagents intended for other tests, such as the prothrombin time activator Innovin or Recombiplastin®, combined with customer-made CaCl2 solution and drugs, such as ReoPro® (abciximab) and Trasylol® (aprotinin). This leads to a low standardization, many pipetting steps and many sources of error.

There is a reagent system for viscoelastic measurements marketed by Pentapharm, which is based on standardized reagents, most of which are provided to the customer in a liquid form, which are pipetted by the user into the test cup using standardized operating procedures. This standardizes the application; however it still requires several pipetting steps for the analysis. For example, to perform a FIBTEM test together with an EXTEM control test, the pipetting of blood, CaCl₂ solution, extrinsic activator and a platelet inhibitor may result in the performance of a total of 8 pipetting steps (including three times changing of the tip during one test procedure) and the need for 3 different reagents that have to be handled by the user. This provides a requirement for training consumes time and is a potential source of error.

There are other reagent systems on the market, which are based on a variety of reagents. Some of them are liquid, and have to be pipetted into the cup (e.g. CaCl₂ solution), some are dried into the test cup (such as heparinase) and some are provided in small vials, in a quantity intended for one test. A characteristic of these reagents is that still each reagent is typically provided alone, and therefore several steps are required at least for tests requiring more than one active reagent.

Accordingly, some efforts have been done in the past to provide a simpler reagent system for viscoelastic measurements of blood or blood components:
- drying them directly into the cup which takes the volume of the sample during measurement
- compound stable liquid combinations of the reagents in the working concentration

One shortcoming of the strategy of drying active reagents directly into the test cup is that these are typically made of plastic and are light, which makes the filling of these cups in automated reagent dispensing lines more difficult. This makes manual filling steps necessary or the development of specialized equipment, which are both costly.

Another shortcoming of this strategy is that the active components or stabilizers may interfere with the adhesion strength of the blood clot on the cup surfaces, which is required to perform correct measurements. For example, there has been shown diminished clot adhesion after incubation of albumin solution into the cup (Albumin is a typical stabilizer used to stabilize all kinds of proteins in reagents):

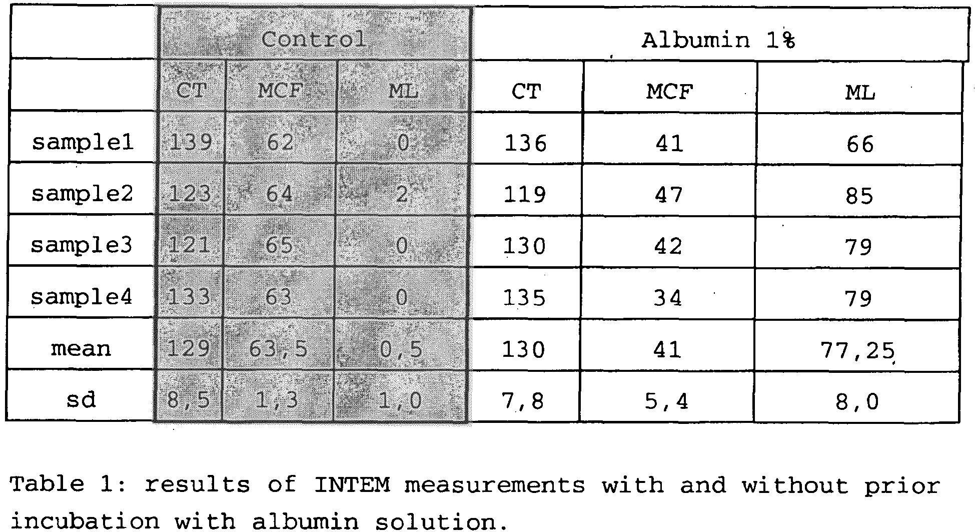

Another possible strategy to simplify the handling of the reagents is to combine the different reagents necessary for one test in liquid phase in their working concentration. The main problem here is the interaction of the different substances while staying together for a longer period. Some components negatively affect the stability of each other when staying together in the liquid phase at higher concentrations; for example, CaCl₂ disturbs the stability of Tissue Factor reagent in liquid phase over the time.

Moreover, if these combined reagents should be provided in an amount sufficient for exactly one test, another problem would arise: In this case, the very small portion of liquid reagent might stick to parts of the reagent container or the cap and might thus not mix sufficiently with the test liquid when the analysis is performed.

One strategy proposed to solve the named aspects was disclosed by Kolde et al. in US patent application 20040071604. In this application, a cup system for viscoelastic analyses is presented, in which the lower end of the cup is divided in several reagent chambers. This allows to place the reagents independently into the different chambers, without mixing them and then to freeze-dry the reagents.

However, disadvantages of this solution include the need for a very precise pipetting process, as the separate reagent chambers are very small and also the problem that the reagent drops might still mix before the freeze-drying by vibrations present on the reagent filling line. Another problem is the possible air-drying of the small reagent drops during the processing under room conditions before the lyophilisation process starts. Again the problem of automatically handling the small plastic and thus very light using standard reagent-filling lines is present.

The most coagulation test methods in routine lab use just measure the time from adding an activator to the sample until the first initial formation of a fibrin clot can be detected (= clotting time). They stop at this point and no further measurement is done. This has the implications, that in these methods a firm adhesion of the blood to the surfaces of the measurement cell is not necessary. Accordingly, the variety of analyzers and reagents available for the assessment of clotting times with such methods does not have to handle the unique problems connected with viscoelastic measurements.

### SUMMARY OF THE INVENTION

Therefore, it is an object of the present invention to provide a container comprising a diagnostic composition, which allows for a safe, reproducible and easy to use procedure for different tests in viscoelastic systems. It is a further object of the present invention to provide a container comprising a diagnostic composition which is specifically adapted to one single analysis of a blood sample and has a superior reagent stability regarding prior art compositions. It is a still further object of the present invention to provide a diagnostic method which provides reliable and reproducible results, is easy to handle and which provides a standardized system for the determination of the coagulation characteristics of a blood sample.

These objects are attained by the container of claim 1, the diagnostic method of claim 8 and the use of claim 14.

Preferred embodiments are set forth in the dependent claims.

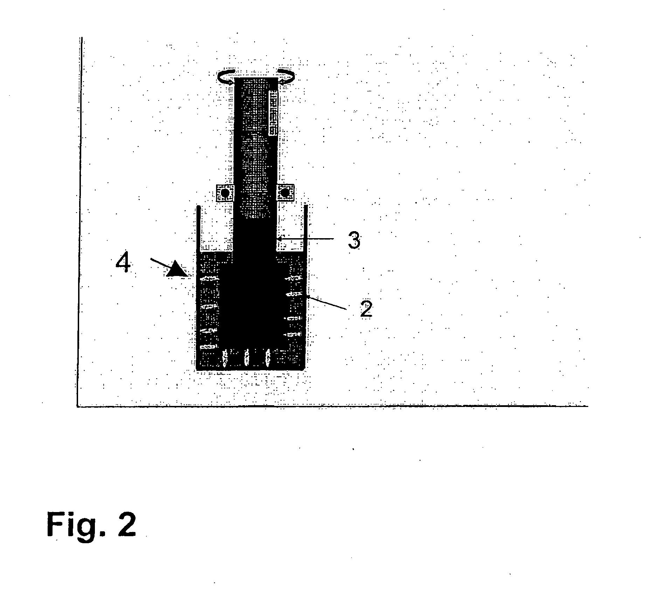

By using the container comprising a diagnostic composition of the invention, the tests may be performed as follows: a defined volume of a sample (e.g., whole blood, blood plasma etc.) is added directly into a container 1 containing the diagnostic composition. After dissolving of the composition in the blood sample, the resulting mixture is pipetted from the container 1 into the measuring cup 2 of a measuring apparatus 4. The cup 2 is then put into a position such that the pin 3 is immersed into the liquid in the test cup (cp. Fig. 2).

Therefore, the user needs only 4 pipetting steps for each test to perform (in the prior art liquid system up to 8 steps, see above) and no change of the pipette tip is necessary.

Thus, it is clear that the present system for the determination of coagulation characteristics of a blood sample can be handled easier, thereby making the likelihood of errors smaller, which can be due to an imprecise line of action by a (potentially less experienced) operator.

Therefrom, further advantages may arise as, for example, a higher reproducibility of the results to be achieved, and thus, a higher degree of standardization.

Furthermore, one advantage of the present approach is that the diagnostic composition (i.e. reagent mixture) may be provided in a larger volume (and thus in a lower concentration) prior to the step of lyophilization than it is present in the final measuring step. In more detail, it is advantageous to initially provide a low concentration of the reagent mixture in order to avoid an early reaction of the reagents. The final concentration in the measuring step (obtained by resolving the diagnostic composition in the test sample) then will be higher than the initial concentration present before the lyophilization step.

### BRIEF DESCRIPTIONS OF THE DRAWINGS

Further features and advantages of the present invention will be evident from a description of embodiments with reference to the figures.

In the figures:

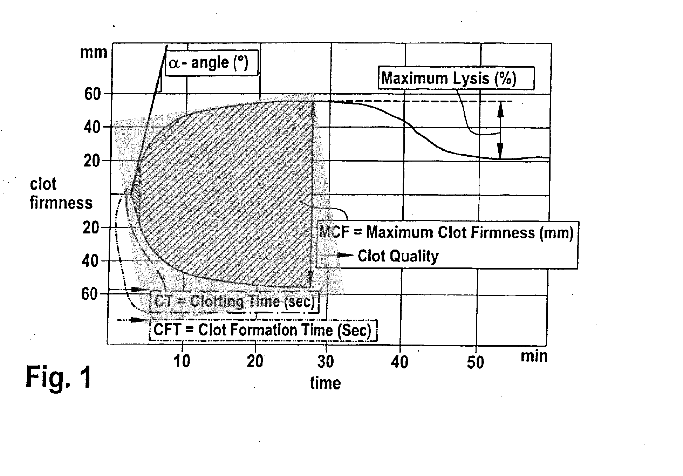

Figure 1 is an exemplary diagram showing a typical thromboelastometric measurement;
Figure 2 is showing a measuring apparatus 4 for thromboelastometric analysis;
Figure 3 is an illustration of a measuring cup 2 of a measuring apparatus 4 of the prior art;
Figure 4A, B, C are schematic cross-sectional views of three preferred embodiments of a container 1 of the present invention.

### DETAILED DESCRIPTION OF THE INVENTION

In a first aspect, the present invention provides a container comprising a diagnostic composition for use in the viscoelastic analysis of a test liquid, comprising the following constituents:
a) at least one activator of coagulation;
b) a calcium salt in an amount sufficient to ensure recalcification of the test liquid, and
c) optionally one or more inhibitors and/or other coagulation components or factors;
characterized in that
the composition is present as an essentially dry mixture of all constituents and in an amount sufficient for performing one single viscoelastic analysis of a specified test liquid.

The calcium salt preferably is CaCl₂.

The diagnostic composition or reaction mixture comprises constituents, which are per se known in the art. One difference to the prior art approaches is, however, that the mixture of these constituents is provided in an essentially dry form.

"Essentially" dry in the context of the invention means a state, wherein the mixture is essentially free from any liquid or moisture, in particular being depleted of water. Water or any other liquid, however, may be present as residue in the mixture, but only to an extent, which does not negatively influence the stability of the overall composition. In particular, it has to be excluded that an interaction occurs between the different constituents, which negatively affects the stability. A remaining amount of liquid, preferably water, in the composition of up to 10 % by weight should be acceptable.

The amount sufficient for performing one single viscoelastic analysis of a specified test liquid, for example a blood sample, is that amount of all constituents in mixture, which provides the required concentration of the reagents in the final analysis of the coagulation characteristics of the blood sample, i.e. in the cup 2 of a measuring apparatus 4. Therefore, it is not necessary to further portion the diagnostic composition before or after dissolving it in a liquid.

Further, this is achieved by dissolving the substances in the test liquid (blood sample etc.) itself, not by dissolving the constituents in an amount of liquid diluent leading to the final working concentration.

The activator of coagulation as mentioned above preferably is an intrinsic and/or extrinsic activator.

The extrinsic activator of coagulation in turn preferably is the Tissue Factor (TF) and is more preferably selected from lipidated TF or rTF.

The intrinsic activator of coagulation is preferably selected from celite, ellagic acid, sulfatit, kaolin, silica, RNA, or mixtures thereof.

As a second feature, the diagnostic composition comprises a calcium salt such as CaCl₂, wherein CaCl₂ is present in an amount of about 1-100 µmol/ml of test liquid. As mentioned above, the amount of CaCl₂ must be sufficient to ensure recalcification of the test liquid, in particular of the blood sample, if the sample was decalcified before. It turned out that the amount of from 3-30 µmol/ml is optimal to achieve this requirement. In order to determine the required amount of CaCl₂ to be contained in the diagnostic composition even more precisely, the exact volume of the test liquid to be collected from the patient has to be known as well as the amount of decalcifying reagent employed.

The diagnostic composition optionally contains one ore more inhibitors, being selected, for example, from one or more of a platelet inhibitor, fibrinolysis inhibitor, or heparin inhibitor.

Those inhibitors may be used and combined depending on the precise diagnostic demands, for example, the platelet inhibitor may be a cyto-skeletton inhibitor or a GPIIb/IIIa antagonist. The like, the fibrinolysis inhibitor can be selected, for example, from aprotinine, tranexamic acid, or eaca; the heparin inhibitor might be selected, for example, from heparinase, protamine or protamine-related peptides; and the coagulation factor can be selected, for example, from one or more coagulation factors or activated coagulation factors preferably FXa or FVa or activated protein C or FVIIa. However, it is noted that this is only a preferred selection and further inhibitors can be used if required.

In a preferred embodiment, the dry mixture is a lyophilized mixture, more preferably a mixture produced by co-lyophilization of a mixture of the liquid reagents in one single lyophilizytion process. More precisely, in this preferred embodiment the diagnostic composition is produced by filling liquid components - in the required quantities to reach the intended composition - into a suitable container 1 (for example by a pipetting machine) and drying this composition in the container 1 under application of low pressure environment (about 1000 - 0.2 mbar) at suitable temperature (about +30°C to -70°C). The container 1 then may be closed or sealed by a close cover or the like (e.g., a lid 5) in order to avoid a loss of reagents, or an invasion of contaminants, water etc.

In a preferred embodiment, the diagnostic composition may also contain one or more stabilizers, wherein the stabilizer preferably is albumin or gelatine.

In a preferred embodiment, the diagnostic composition may also contain one or more phospholipids, wherein the phospholipids may be a composition of different phospholipids like phosphatidyserine, phosphatidylethanolamine and phosphatidylethanolcholine. For example, mixtures of phospholipids extracted from rabbit brain may be used.

The present diagnostic composition may have the following constitution in preferred embodiments:
- extrinsic activation: Combination of extrinsic activator and stabilizer and, optionally, CaCl₂
- intrinsic activation: Combination of intrinsic activator and stabilizer and, optionally, CaCl₂
- extrinsic activation insensitive for heparin: Combination of extrinsic activator, heparin inhibitor and stabilizer and, optionally, CaCl₂
- intrinsic activation insensitive for heparin: Combination of intrinsic activator, heparin inhibitor and stabilizer and, optionally, CaCl₂
- extrinsic activation without platelet activation: Combination of extrinsic activator, platelet inhibitor and stabilizer and, optionally, CaCl₂
- extrinsic activation without platelet activation, insensitive for heparin: Combination of extrinsic activator, platelet inhibitor, heparin inhibitor and stabilizer and, optionally, CaCl₂
- intrinsic activation without platelet activation: Combination of intrinsic activator, platelet inhibitor and stabilizer and, optionally, CaCl₂
- intrinsic activation without platelet activation, insensitive for heparin: Combination of intrinsic activator, platelet inhibitor, heparin inhibitor and stabilizer and, optionally, CaCl₂
- extrinsic activation with inhibition of fibrinolysis: Combination of extrinsic activator, fibrinolysis inhibitor and stabilizer and, optionally, CaCl₂
- extrinsic activation with inhibition of fibrinolysis, insensitive for heparin: Combination of extrinsic activator, fibrinolysis inhibitor, heparin inhibitor and stabilizer and, optionally, CaCl₂
- intrinsic activation with inhibition of fibrinolysis: Combination of intrinsic activator, fibrinolysis inhibitor and stabilizer and, optionally, CaCl₂
- intrinsic activation with inhibition of fibrinolysis, insensitive for heparin: Combination of intrinsic activator, fibrinolysis inhibitor, heparin inhibitor and stabilizer and, optionally, CaCl₂
- extrinsic activation with additional coagulation factor: Combination of extrinsic activator, one additional coagulation factor and stabilizer and, optionally, CaCl₂
- extrinsic activation with additional coagulation factor, insensitive for heparin: Combination of extrinsic activator, one additional coagulation factor, heparin inhibitor and stabilizer and, optionally, CaCl₂
- intrinsic activation with additional coagulation factor: Combination of intrinsic activator, one additional coagulation factor and stabilizer and, optionally, CaCl₂
- intrinsic activation with additional coagulation factor, insensitive for heparin: Combination of intrinsic activator, one additional coagulation factor, heparin inhibitor and stabilizer and, optionally, CaCl₂

The present invention provides a container 1, comprising the diagnostic composition as defined above. The container 1 preferably takes the form of a vial or a cuvette.

The container 1 is formed from a material (e.g., plastic or glass) which is not corroded or otherwise affected by the reagents to be filled in or the test liquid to be filled in.

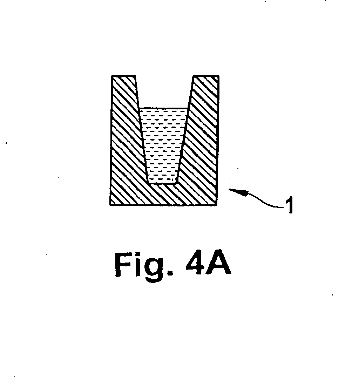

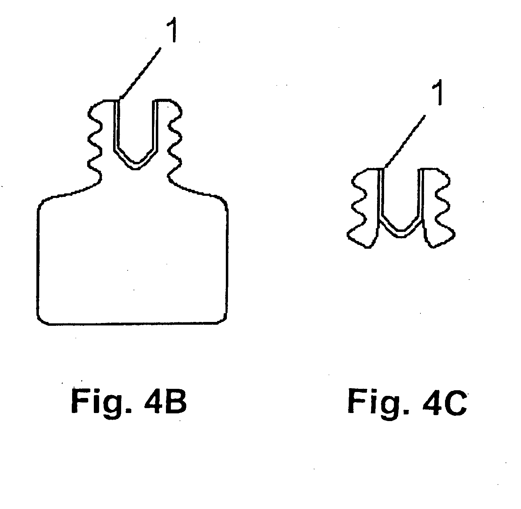

The container 1 may have cylindrical shape, but its shape does not necessarily have to be cylindrical. The container 1 may have a form which reduces its inner lateral profile from the upper opening to the bottom, as for example a conically shaped form as indicated in Fig. 4 or at least a partially conical form. This provides a better handling of the usually small amounts of liquid reagent mixture: In a flat bottom container, for example in a common vial with similar diameter, the used amount of liquid would hardly cover the whole bottom and might dry in an uncontrolled manner before the lyophilization process starts. Using flat bottom vials with a smaller diameter would reduce this problem, but such vials might then have an opening diameter which is too narrow to be handled with standard pipetting equipment used in connection with diagnostic devices like, for example, the ROTEM thromboelastometer. (Regarding the design of the ROTEM system and how to use it, it is referred to the publication of US 5,777,215).

Furthermore, such small vials might become harder to manage for common automated processing systems and might thus increase the production costs.

The cross-section of a basically axially symmetric container 1 of a preferred embodiment is shown in Fig. 4A. However, it should be explicitly noted that the present invention is not restricted thereto, and also U-shaped, rectangular shaped or the like forms may be used.

As mentioned above, the container 1 may be closed or sealed by a lid 5 or the like in order to avoid a loss of reagents, or an invasion of contaminants, water etc.

In a further embodiment, the container 1 is designed in a way that it can be directly used as the measuring cup 2 of the viscoelastic measuring apparatus 4. In other words, a viscolelastic analysis can be performed such that a respective container 1 is provided, the test liquid is added into the container 1, and the measurement is performed directly in the container 1. In this case, only the blood dispension into the container has to be performed as a liquid transfer step, which can be realized by using a manual pipette, an automated pipette, an automated dispenser or any other liquid transfer equipment.

In this embodiment, the container 1 might be designed by combination of two materials, e.g., glass and plastic or glass and a surface covering. As indicated in Figure 4B, this combination can be realized by providing a glass container with a plastic insert forming the portion where the liquid reagent is filled in before lyophilization and where the test liquid is added to before the viscoelastic measurement. In this context the part of the container made from glass does not necessarily have to clasp the entire underside of the plastic part but might be constructed according to Figure 4C. In Figure 4B and C, a further embodiment of the invention can be seen. The container 1 (for example a cuvette) may be incorporated in a larger structure, for example a glass article, which provides some technical advantages: at first, thus set-up may technically facilitate the step of lyphilisation, and secondly, may provide a holding for the container.

In these embodiments, possible coagulation activation in the test liquid by the glass surface is excluded, while the superior sealing properties of the glass material when compared to plastic material are still used. The similar effect of suppressing possible coagulation activation in the test liquid by the glass surface can be realized by covering the glass surface (or at least the inner portion of the glass surface) with a layer of one or more substances that are not able to activate coagulation if they are in contact with blood or blood components.

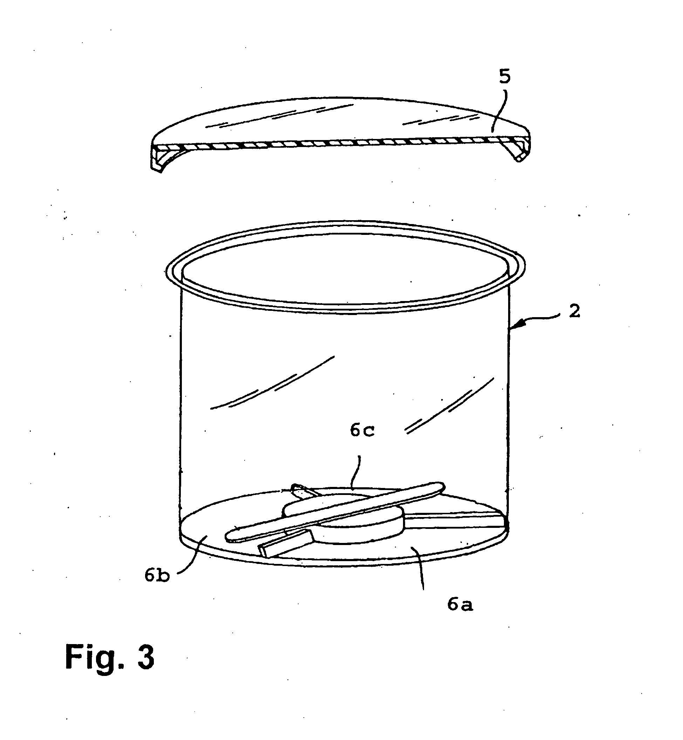

For comparison, a prior art measuring cup 2 according to US 2004/0071604 is illustrated in Fig. 3:
A container 1 is provided, which serves as reagent support and measuring vessel (i.e. can be regarded as a measuring cup 2) for analysis using various analytical processes, and has a region which is divided into at least two chambers (6a, 6b, 6c) by one or more bars extending from the container wall or the container base, wherein the chambers are arranged so that liquid or solid reagents may be introduced therein without them being able to be mixed by diffusion or running into one another. The container 1 is used for drying or freeze-drying by with completely or partly filled chambers and serves at the same time as a measuring vessel after re-dissolving the dried material by adding water, reagent solution, or the sample present in aqueous phase.

Hence, the main difference of the present invention when compared to prior art in US 2004/0071604 is provided by the co-lyphylization process of a mixture of reagents in the cup. This makes the division of the vessel into two or more regions - separated by bars - unnecessary, thus avoiding the resulting complications for the production process by filling in at least two different liquids into two different portions of the vessel.

In a second aspect, the present invention is directed to a method of performing a viscoelastic analysis on a test liquid, a blood sample, comprising the steps of:
a) providing a container 1 as defined above;
b) adding the test liquid into said container 1, thereby dissolving the diagnostic composition contained therein;
c) transferring the mixture of said test liquid and said diagnostic composition into a cup 2 and put it into an apparatus 4 suitable for performing a viscolelastic analysis, or putting the container 1 into an apparatus 4 suitable for performing a viscolelastic analysis; and
d) performing the viscoelastic analysis of said mixture.

As already mentioned above, the test liquid preferably is a blood sample, preferably is a mammalian, more preferably a sample of human blood or blood components (e.g., whole blood or blood plasma).

Step b) of the method of the present invention preferably takes about 1-60, more preferably 2-10 sec and most preferred is about 5 sec. Following that time, the mixture of the diagnostic composition and the blood sample should be quickly transferred to the measuring cup 2 of the measuring apparatus 4. This is done in step d) by manually or automatically pipetting the mixture from the container 1 and by transferring it thereby to the apparatus 4, i.e. to the measuring cup 2 of the apparatus 4.

As an alternative, if the container 1 of the present invention is the measuring cup 2, the measurement is performed directly in the container 1. In this case, step c) may be omitted. The apparatus 4 preferably is a device suited for viscoelastic measurements, for example devices disclosed in (US 5,777,215), (US 6,537,819), or (US 5,777,215).

One example of that apparatus 4 is shown in Fig. 2:
After the formation of the clot between cup 2 (cuvette) and pin 3, the clot itself is stretched by the movement of the pin 3 relative to the cup 2. The detection of the characteristic parameters of the clot is based on the mechanical coupling of cup 2 and pin 3 by the clot. This is only possible if the clot adheres on the surfaces of both cup 2 and pin 3. So, a firm adhesion on the surfaces of both cup 2 and pin 3 is essentially required for the viscoelastic analysis.

The method of the present invention comprises such a viscoelastic analysis of a blood sample in order to determine its coagulation characteristics, wherein such a viscoelastic analysis in the broadest sense is the measurement of a relative movement of a cuvette containing a blood sample relative to a punch. The analysis preferably comprises the determination of the clotting time, the clot formation time, and the firmness of the clot over time including fibrinolytic effects.

In practice, the following steps may be performed:
1. a defined volume of a sample (e.g., whole blood, plasma) is added directly into a vial containing the lyophilized reagent composition; the measurement should start at a time close to the moment of adding the sample
2. after dissolving of the reagent mixture in the sample (5 sec.) the reagent-sample mixture is pipetted from the reagent vial into the measuring cup 2 (not necessary if the vial functions itself as measuring cup 2)
3. the cup 2 is then put into a position such that the pin 3 is immersed into the liquid contained in the test cup, the measurement continues until stopped by the user

Therefore, the user needs not more than 4 pipetting steps in total for each test to perform (compared to the up to 8 steps required for a liquid reagent system) and no change of pipette tip is necessary when preparing one test. This clearly indicates the direct benefit of the present invention for the person who is performing such tests.

In a further aspect, the present invention is directed to the use of a container 1 as defined above in a method for analyzing the viscoelastic behaviour of a test liquid, preferably a blood sample.

Although the present invention has been described in accordance with preferred embodiments, it is obvious for a person skilled in the art that modifications are possible in all embodiments.

### Reference signs

- 1: container
- 2: measuring cup
- 3: pin
- 4: measuring apparatus
- 5: lid
- 6 a, b, c: reagent chambers

In the following some preferred embodiments of a diagnostic composition are described:
A) A diagnostic composition for use in the viscoelastic analysis of a test liquid, comprising the following constituents: a) at least one activator of coagulation; b) a calcium salt, preferably CaCl2, in an amount sufficient to ensure recalcification of the test liquid; and, c) optionally one or more inhibitors and/or other coagulation components or factors; wherein the composition is present as an essentially dry mixture of all constituents and in an amount sufficient for performing one single viscoelastic analysis of a specified test liquid.
B) The diagnostic composition of embodiment A), wherein the activator of coagulation is an intrinsic and/or extrinsic activator.
C) The diagnostic composition of embodiment A) or B) wherein the extrinsic activator of coagulation is the Tissue Factor (TF).
D) The diagnostic composition of embodiment C), wherein the Tissue Factor is selected from lipidated TF or rTF.
E) The diagnostic composition of embodiment A) or B), wherein the intrinsic activator of coagulation is selected from the group consisting of celite, ellagic acid, sulfatit, kaolin, silica, RNA, or mixtures thereof.
F) The diagnostic composition of one of embodiments A) to E), wherein the inhibitor is selected from one or more of a platelet inhibitor, fibrinolysis inhibitor, or heparin inhibitor.
G) The diagnostic composition of embodiment F), wherein platelet inhibitor is a cytoskeletton inhibitor or a GPIIb/IIIa antagonist.
H) The diagnostic composition of embodiment F), wherein the fibrinolysis inhibitor is selected from aprotinine, tranexamic acid, or eaca.
J) The diagnostic composition of embodiment F), wherein the heparin inhibitor is selected from heparinase, protamine or protamine-related peptides.
K) The diagnostic composition of one of embodiments A) to J), wherein the coagulation factor is selected from one or more coagulation factors or activated coagulation factors, preferably FXa or FVa or activated protein C or FVIIa.
L) The diagnostic composition of one of embodiments A) to K), wherein CaCl2 is present in an amount of about 1-100 µmol/ml of the test liquid, preferably the blood sample.
M) The diagnostic composition of one of embodiments A) to L), wherein the dry mixture is a lyophilized mixture.
N) The diagnostic composition of embodiment M), wherein the lyophilized mixture is prepared by lyophilizing a liquid mixture of the contained components in a single process.
O) The diagnostic composition of one of embodiments A) to N), which further comprises a stabilizer.
P) The diagnostic composition of one of embodiments A) to O), wherein the stabilizer is albumin or gelatine.
   Preferred embodiments of a container according to the invention are:
Q) A container, comprising the diagnostic composition of one or more of preferred embodiments A) to P).
R) The container of embodiment Q), wherein the container 1 takes the form of a vial or a cuvette.
S) The container of embodiment R), which is formed in a way that the inner lateral profile reduces from the opening to the bottom.
T) The container of embodiment Q), R), or S), with its inner portion shaped in a manner that it can be attached to a device for performing viscoelastic measurements.
U) The container of embodiment T) consisting of at least two different materials, where one material forming basically or at least partially the outer shape of the container enables hermetical sealing of the container by a suited cover, while the other material basically covering the portion conceiving the test liquid enables proper adhesion of blood or blood components without activating the coagulation cascade.
   Some preferred variants of a method according to the invention are discussed in the following:
V) A method of performing a viscoelastic analysis on a test liquid, comprising the steps of: a) obtaining a test liquid; b) providing a container 1 according to one of the preferred embodiments Q) to U); c) adding the test liquid into said container 1, thereby dissolving the diagnostic composition contained therein; d) optionally transferring the mixture of said test liquid and said diagnostic composition into an apparatus 4 suitable for performing a viscolelastic analysis; or putting the container 1 into an apparatus 4 suitable for performing a viscolelastic analysis; and e) performing the viscoelastic analysis of said mixture.
W) The method of variant V), wherein the test liquid is a blood sample, preferably a mammalian, more preferably a human blood sample.
X) The method of variant W), wherein the blood sample is whole blood or blood plasma.
Y) The method of one or more of variants V) to X), wherein step c) takes about 1-60 sec., preferably about 2-10 sec., more preferably about 5 sec.
Z) The method of one or more of variants V) to Y), wherein the mixture is transferred in step d) by manually or automatically pipetting the mixture from the container 1 and by transferring it thereby to the apparatus 4.
Z.a) The method of one or more of variants V) to Z), wherein the mixture is transferred to the measuring cup 2 of the apparatus 4.
Z.b) The method of one or more of variants V) to Z.a), wherein the apparatus 4 is a thromboelastometer or a thrombelastograph.
Z.c) The method of one or more of variants V) to Z.b), wherein the analysis comprises the determination of the clotting time, the clot formation time, the firmness of the clot over time and/or fibrinolysis.

It is a preferred to use a container 1 of one or more of the preferred embodiments Q) to U) in a method for analyzing the viscoelastic behaviour of a test liquid.

It is specially preferred to use at least two containers 1 of one or more of the preferred embodiments Q) to U) in a combined method for analyzing the viscoelastic behaviour of the same test liquid.

## Claims

1. A container (1), suitable to conceiving a defined volume of a blood sample, wherein the container contains a lyophilized diagnostic composition for use in the viscoelastic analysis of the blood sample, the lyophilized diagnostic composition comprising the following constituents:
a) at least one activator of coagulation;
b) a calcium salt, in an amount of 1-100 µmol/ ml of the blood sample, sufficient to ensure recalcification of the blood sample;
c) optionally one or more inhibitors, wherein said one or more inhibitors are selected from the group consisting of a platelet inhibitor, a fibrinolysis inhibitor, and a heparin inhibitor; and
d) optionally one or more coagulation factors or activated coagulation factors, wherein said one or more coagulation factors or activated coagulation factors are selected from the group consisting of FI, FII, FIII, FIV, FV, FVI, FVII, FVIII, FIX, FX, FXI, FXII, FXIII, FIa, FIIa, FIIIa, FIVa, FVa, FVIa, FVIIa, FVIIIa, FIXa, FXa, FXIa, FXIIa, FXIIIa, and activated protein C;
wherein the container (1) has the form which reduces its inner lateral profile from the upper opening to the bottom and wherein the container (1) is designed by either glass and plastic or glass and a surface covering, said surface covering being a layer of one or more substances that are not able to activate coagulation if they are in contact with blood or blood components.

2. The container according to claim 1, wherein the sample is whole blood or plasma.

3. The container according to claim 1 or 2, wherein the activated coagulation factors are selected from FXa, FVa, activated protein Cor FVIIa.

4. The container according to any one of claims 1 to 3 which takes the form of a vial or a cuvette.

5. The container according to any one of claims 1 to 4, wherein in the lyophilized diagnostic compostion one activator of coagulation is an extrinsic activator.

6. The container according to any one of claims 1 to 4, wherein in the lyophilized diagnostic composition one activator of coagulation is an intrinsic activator.

7. The container according to any one of claims 1 to 6, wherein the lyophilized diagnostic composition comprises a stabilizer.

8. A method of performing a viscoelastic analysis on a blood sample comprising the steps of:
a) providing a container (1) according to any of claims 1 to 7;
b) adding the blood sample into said container (1), thereby dissolving the diagnostic composition contained therein;
c) optionally transferring the mixture of said blood sample and said diagnostic composition into an apparatus (4) suitable for performing a viscoelastic analysis; or putting the container (1) into an apparatus (4) suitable for performing a viscoelastic analysis; and
d) performing the viscoelastic analysis of said mixture.

9. The method according to claim 8, wherein step b) takes 1-60 sec.

10. The method according to claim 8 or 9, wherein the mixture is transferred in step c) by manually or automatically pipetting the mixture from the container (1) and by transferring it thereby to the apparatus (4).

11. The method according to any one of claims 8 to 10, wherein the mixture is transferred to the measuring cup (2) of the apparatus (4).

12. The method according to any one of claims 8 to 11, wherein the apparatus (4) is a thromboelastometer or a thrombelastograph.

13. The method according to any one of claims 8 to 12, wherein the analysis comprises the determination of the clotting time, the clot formation time, the firmness of the clot over time, and/ or fibrinolysis.

14. A use of a container (1) according to any of claims 1 to 7 in a method for analyzing the viscoelastic behavior of a blood sample.

15. The use according to claim 14, wherein at least two containers (1) according to any of claims 1 to 7 are used in a combined method for analyzing the viscoelastic behavior of the same blood sample.

## Patentansprüche

1. Behälter (1), geeignet zum Aufnehmen eines definierten Volumens einer Blutprobe, wobei der Behälter eine lyophilisierte diagnostische Zusammensetzung für die Verwendung bei der viskoelastischen Analyse der Blutprobe enthält, w o b e i d i e lyophilisierte diagnostische Zusammensetzung folgende Bestandteile umfasst:
a) wenigstens einen Koagulationsaktivator;
b) ein Calciumsalz in einer Menge von 1-100 µmol/ml der Blutprobe, ausreichend zum Gewährleisten von Rekalzifikation der Blutprobe;
c) gegebenenfalls einen oder mehrere Inhibitoren, wobei der eine oder die mehreren Inhibitoren ausgewählt sind aus der Gruppe bestehend aus einem Plättcheninhibitor, einem Fibrinolyseinhibitor und einem Heparininhibitor; und
d) gegebenenfalls einen oder mehrere Koagulationsfaktoren oder aktivierte Koagulationsfaktoren, wobei der eine oder die mehreren Koagulationsfaktoren oder aktivierten Koagulationsfaktoren ausgewählt sind aus der Gruppe bestehend aus FI, FII, FIII, FIV, FV, FVI, FVII, FVIII, FIX, FX, FXI, FXII, FXIII, FIa, FIIa, FIIIa, FIVa, FVa, FVIa, FVIIa, FVIIIa, FIXa, FXa, FXIa, FXIIa, FXIIIa und aktiviertem Protein C;
wobei der Behälter (1) eine Form aufweist, die sein inneres seitliches Profil von der oberen Öffnung zu dem Boden des Behälters verringert und wobei der Behälter (1) entweder aus Glas und Kunststoff oder Glas und einer Oberflächenbeschichtung aufgebaut ist, wobei die Oberflächenbeschichtung eine Schicht aus einem oder mehreren Stoffen ist, die nicht fähig sind, Koagulation zu aktivieren, wenn sie in Kontakt mit Blut oder Blutkomponenten stehen.

2. Behälter gemäß Anspruch 1, wobei die Probe Vollblut oder Plasma ist.

3. Behälter gemäß Anspruch 1 oder 2, wobei die aktivierten Koagulationsfaktoren ausgewählt sind aus FXa, FVa, aktiviertem Protein C und FVIIa.

4. Behälter gemäß einem der Ansprüche 1 bis 3, der die Form eines Fläschchens oder einer Küvette aufweist.

5. Behälter gemäß einem der Ansprüche 1 bis 4, wobei in der lyophilisierten diagnostischen Zusammensetzung ein Koagulationsaktivator ein extrinsischer Aktivator ist.

6. Behälter gemäß einem der Ansprüche 1 bis 4, wobei in der lyophilisierten diagnostischen Zusammensetzung ein Koagulationsaktivator ein intrinsischer Aktivator ist.

7. Behälter gemäß einem der Ansprüche 1 bis 6, wobei die lyophilisierte diagnostische Zusammensetzung einen Stabilisator umfasst.

8. Verfahren zum Durchführen einer viskoelastischen Analyse einer Blutprobe, umfassend die Schritte:
a) Bereitstellen eines Behälters (1) gemäß einem der Ansprüche 1 bis 7;
b) Zugeben der Blutprobe in den Behälter (1), um dadurch die darin enthaltene diagnostische Zusammensetzung zu lösen;
c) gegebenenfalls Überführen des Gemischs der Blutprobe und der diagnostischen Zusammensetzung in eine Vorrichtung (4), die zum Durchführen einer viskoelastischen Analyse geeignet ist; oder Anordnen des Behälters (1) in einer Vorrichtung (4), die zum Durchführen einer viskoelastischen Analyse geeignet ist; und
d) Durchführen der viskoelastischen Analyse des Gemischs.

9. Verfahren gemäß Anspruch 8, wobei Schritt b) 1-60 sec benötigt.

10. Verfahren gemäß Anspruch 8 oder 9, wobei das Gemisch bei Schritt c) überführt wird, indem das Gemisch manuell oder automatisch aus dem Behälter (1) pipettiert und so in die Vorrichtung (4) überführt wird.

11. Verfahren gemäß einem der Ansprüche 8 bis 10, wobei das Gemisch in den Messtiegel (2) der Vorrichtung (4) überführt wird.

12. Verfahren gemäß einem der Ansprüche 8 bis 11, wobei die Vorrichtung (4) ein Thromboelastometer oder ein Thromboelastograph ist.

13. Verfahren gemäß einem der Ansprüche 8 bis 12, wobei die Analyse das Bestimmen der Gerinnungszeit, der Gerinnselbildungszeit, der Festigkeit des Gerinnsels über die Zeit und/oder der Fibrinolyse umfasst.

14. Verwendung eines Behälters (1) gemäß einem der Ansprüche 1 bis 7 bei einem Verfahren zum Analysieren des viskoelastischen Verhaltens einer Blutprobe.

15. Verwendung gemäß Anspruch 14, wobei wenigstens zwei Behälter (1) gemäß einem der Ansprüche 1 bis 7 bei einem kombinierten Verfahren zum Analysieren des viskoelastischen Verhaltens der gleichen Blutprobe verwendet werden.

## Revendications

1. Récipient (1), adapté pour contenir un volume défini d'un échantillon de sang, **caractérisé en ce que** le récipient contient une composition diagnostique lyophilisée pour utilisation dans l'analyse viscoélastique de l'échantillon de sang, la composition diagnostique lyophilisée comprenant les constituants suivants :
a) au moins un activateur de coagulation ;
b) un sel de calcium, en une quantité de 1 à 100 µmol/ml de l'échantillon de sang, suffisante pour assurer la recalcification de l'échantillon de sang ;
c) facultativement un ou plusieurs inhibiteurs, lesdits un ou plusieurs inhibiteurs étant choisis dans le groupe constitué d'un inhibiteur plaquettaire, un inhibiteur de fibrinolyse et un inhibiteur d'héparine ; et
d) facultativement un ou plusieurs facteurs de coagulation ou facteurs de coagulation activés, lesdits un ou plusieurs facteurs de coagulation ou facteurs de coagulation activés étant choisis dans le groupe constitué de FI, FII, FIII, FIV, FV, FVI, FVII, FVIII, FIX, FX, FXI, FXII, FXIII, FIa, Fila, FIIIa, FIVa, FVa, FVIa, FVIIa, FVIIIa, FIXa, FXa, FXIa, FXIIa, FXIIIa, et la protéine C activée ;
le récipient (1) ayant la forme qui réduit son profil latéral interne de l'ouverture supérieure à la base et le récipient (1) étant fabriqué en verre et en plastique ou en verre et une couverture de surface, ladite couverture de surface étant une couche d'une ou plusieurs substances qui ne sont pas capables d'activer la coagulation si elles sont en contact avec du sang ou des composants sanguins.

2. Récipient selon la revendication 1, **caractérisé en ce que** l'échantillon est du sang total ou du plasma.

3. Récipient selon la revendication 1 ou 2, **caractérisé en ce que** les facteurs de coagulation activés sont choisis parmi FXa, FVa, la protéine C activée ou FVIIa.

4. Récipient selon l'une quelconque des revendications 1 à 3 qui a la forme d'un flacon ou d'une cuvette.

5. Récipient selon l'une quelconque des revendications 1 à 4, **caractérisé en ce que**, dans la composition diagnostiquée lyophilisée, un activateur de coagulation est un activateur extrinsèque.

6. Récipient selon l'une quelconque des revendications 1 à 4, **caractérisé en ce que**, dans la composition diagnostiquée lyophilisée, un activateur de coagulation est un activateur intrinsèque.

7. Récipient selon l'une quelconque des revendications 1 à 6, **caractérisé en ce que** la composition diagnostique lyophilisée comprend un stabilisant.

8. Procédé de conduite d'une analyse viscoélastique sur un échantillon de sang comprenant les étapes de :
a) fourniture d'un récipient (1) selon l'une quelconque des revendications 1 à 7 ;
b) ajout de l'échantillon de sang dans ledit récipient (1), de manière à dissoudre la composition diagnostique contenue dans celui-ci ;
c) facultativement, le transfert du mélange dudit échantillon de sang et de ladite composition diagnostique dans un appareil (4) adapté pour effectuer une analyse viscoélastique ; ou le placement du récipient (1) dans un appareil (4) adapté pour effectuer une analyse viscoélastique ; et
d) conduite de l'analyse viscoélastique dudit mélange.

9. Procédé selon la revendication 8, dans lequel l'étape b) dure 1 à 60 s.

10. Procédé selon la revendication 8 ou 9, dans lequel le mélange est transféré dans l'étape c) par pipetage manuel ou automatique du mélange à partir du récipient (1) et par transfert de celui-ci dans l'appareil (4).

11. Procédé selon l'une quelconque des revendications 8 à 10, dans lequel le mélange est transféré dans la cuve de mesure (2) de l'appareil (4).

12. Procédé selon l'une quelconque des revendications 8 à 11, dans lequel l'appareil (4) est un thromboélastomètre ou un thromboélastographe.

13. Procédé selon l'une quelconque des revendications 8 à 12, dans lequel l'analyse comprend la détermination du temps de formation de caillot, de la fermeté du caillot au cours du temps et/ou de la fibrinolyse.

14. Utilisation d'un récipient (1) selon l'une quelconque des revendications 1 à 7 dans un procédé pour analyser le comportement viscoélastique d'un échantillon de sang.

15. Utilisation selon la revendication 14, dans laquelle au moins deux récipients (1) selon l'une quelconque des revendications 1 à 7 sont utilisés dans un procédé combiné pour analyser le comportement viscoélastique du même échantillon de sang.
